# EUROPEAN PATENT APPLICATION

(11) **EP 3 042 701 A1**
(43) Date of publication of application: **13.07.2016**
(21) Application number: 14842104.3
(22) Date of filing: 19.08.2014
(51) Int. Cl.: A63B 24/00, A61B 5/0245, A61B 5/083, A61B 5/22, A63B 69/00, G01N 33/497

(54) **EXERCISE LOAD DETERMINATION METHOD AND EXERCISE LOAD DETERMINATION DEVICE**

(30) Priority: 05.09.2013 JP 2013184397
(71) Applicant: NTT Docomo, Inc., Tokyo 100-6150 (JP)
(72) Inventor: TOYOOKA, Tsuguyoshi, Tokyo 100-6150 (JP); HIYAMA, Satoshi, Tokyo 100-6150 (JP); YAMADA, Yuuki, Tokyo 100-6150 (JP); KIMURA, Yutaka, Hirakata-shi Osaka 573-1191 (JP); TSUTSUMI, Hiromi, Hirakata-shi Osaka 573-1191 (JP)
(74) Representative: Viering, Jentschura & Partner mbB Patent- und Rechtsanwälte
(86) International application number: PCT/JP2014/071660
(87) International publication number: WO 2015/033771

(57) **Abstract**

An object is to provide an exercise load determination method and an exercise load determination device that can easily and accurately determine whether an exercise loadis appropriate or not. An acquisition unit (21) acquires first acetone concentration value being concentration of acetone released from a subject before exercise of the subject and second acetone concentration value being concentration of acetone released from the subject after a specified period of time from an end of the exercise by the subject, a determination unit (22) calculates a comparison value between the first acetone concentration value and the second acetone concentration value, compares the comparison value with a preset threshold, and determines whether an exercise load of the exercise is appropriate for the subject, and an output unit (23) outputs information based on a determination result by the determination unit (22).

## Description

### Technical Field

The present invention relates to an exercise load determination method and an exercise load determination device.

### Background Art

In recent years, lifestyle-related diseases caused by daily living habits like diet, smoking and drinking have grown into serious problems. Particularly, obesity that raises the risk of lifestyle-related diseases is often cited as causing numerous diseases, and various measures to prevent or ameliorate obesity have been studied. As a measure to prevent or ameliorate obesity, it is desirable to efficiently bum fat.

Acetone that is released through the expired air and skin is one of metabolic products generated in connection with fat burning and decomposition, and known to be produced in blood and then excreted to the outside of a body as expired gas or skin gas through the lungs or skin. Acetone is significantly effective to objectively grasp a fat burning state, and it is expected as an indicator that specifies the effects of measures to prevent or ameliorate obesity. For example, a method that determines an exercise load such as exercise intensity which is the most appropriate for fat burning by measuring the breath acetone concentration during exercise is disclosed. The exercise load is a load by physical exercise such as sports.

Patent Literature 1 discloses a method that sets the exercise intensity at which the breath acetone concentration starts increasing during exercise to V(%), and determines the exercise intensity V-20(%) to V+30(%) for ordinary people who are not athletes and the exercise intensity V-6.5(%) to V+40(%) for athletes as the exercise intensity which is the most appropriate for fat burning. Further, Patent Literature 2 discloses a method that determines the exercise load which is the most appropriate for fat burning from a rate of change of the breath acetone concentration during exercise when the exercise load changes.

Further, in the fields of sports science and kinesiology, a cardiopulmonary exercise test is conducted with a subject under a doctor's supervision to calculate an anaerobic threshold (AT) and a respiratory compensation point (RC) at which the exercise load which is the most appropriate for fat burning is reached. This cardiopulmonary exercise test measures the concentration of oxygen and carbon dioxide contained in expired air and inspired air during exercise by using a continuous expired gas analysis device.

### Citation List

### Patent Literature

PTL1: Japanese Unexamined Patent Publication No. 2010-268864
PTL2: Japanese Unexamined Patent Publication No. 2012-11133

### Summary of Invention

### Technical Problem

Both of the techniques described in the above-described patent literatures measure expired air during exercise. When measuring the acetone concentration during exercise, there is a case where a difference in exercise load is almost not reflected on the acetone concentration. Specifically, because the techniques described in the above-described patent literatures use a measured value during exercise when the acetone concentration does not significantly change, it is not possible to determine whether variation in the acetone concentration is due to fat burning or just a measurement error. Thus, the techniques described in the above-described patent literatures have a problem that it is difficult to accurately determine the most appropriate exercise load.

Further, in order to conduct the cardiopulmonary exercise test described above, a doctor's supervision is required, and further knowledge and know-how about exercise physiology and a large-sized measurement device like a continuous expired gas analysis device are required, and therefore there is a problem that it is not possible to easily and readily conduct the test by anyone regardless of time and place.

The present invention has been accomplished to solve the above problems and an object of the present invention is thus to provide an exercise load determination method and an exercise load determination device that can easily and accurately determine whether an exercise load is appropriate or not.

### Solution to Problem

An exercise load determination method according to one embodiment of the present invention includes a first acetone concentration acquisition step of acquiring first acetone concentration value being concentration value of acetone released from a subject before exercise of the subject, a second acetone concentration acquisition step of acquiring second acetone concentration value being concentration value of acetone released from the subject after a specified period of time from an end of the exercise by the subject, a determination step of calculating a comparison value between the first acetone concentration value acquired in the first acetone concentration acquisition step and the second acetone concentration value acquired in the second acetone concentration acquisition step, comparing the comparison value with a preset threshold, and determining whether an exercise load of the exercise is appropriate for the subject, and an output step of outputting information based on a determination result in the determination step.

An exercise load determination device according to one embodiment of the present invention includes a first acetone concentration acquisition means configured to acquire first acetone concentration value being concentration value of acetone released from a subject before exercise of the subject, a second acetone concentration acquisition means configured to acquire second acetone concentration value being concentration value of acetone released from the subject after a specified period of time from an end of the exercise by the subject, a determination means configured to calculate a comparison value between the first acetone concentration value acquired by the first acetone concentration acquisition means and the second acetone concentration value acquired by the second acetone concentration acquisition means, compare the comparison value with a preset threshold, and determine whether an exercise load of the exercise is appropriate for the subject, and an output means configured to output information based on a determination result by the determination means.

In the above-described exercise load determination method and exercise load determination device according to one embodiment of the present invention, because the determination is made using the acetone concentration value after a specified period of time has elapsed from the end of the exercise by the subject, which is the timing when fat burning is noticeably reflected on the acetone concentration value, it is possible to accurately determine whether the exercise load is appropriate or not. Further, in the exercise load determination method and the exercise load determination device according to one embodiment of the present invention, because the determination is made based on the acetone concentration measured before and after the exercise of the subject, it is possible to easily determine whether the exercise load is appropriate or not compared with the case of performing a cardiopulmonary exercise test.

Further, in the exercise load determination method according to one embodiment of the present invention, the specified period of time may be set in accordance with the exercise performed or information about a dietary intake condition of the subject. In this case, the second acetone concentration value at the timing when fat burning is reflected on the acetone concentration value can be acquired.

The exercise load determination method according to one embodiment of the present invention may further include a first measurement step of measuring the first acetone concentration value from the subject, and a second measurement step of measuring the second acetone concentration value from the subject, and the first acetone concentration acquisition step may acquire the first acetone concentration value measured in the first measurement step, and the second acetone concentration acquisition step may acquire the second acetone concentration value measured in the second measurement step. In this case, it is possible to reliably acquire the first acetone concentration value and the second acetone concentration value.

In the exercise load determination method according to one embodiment of the present invention, the first acetone concentration value and the second acetone concentration value may be the acetone concentration measured based on a gas component produced from at least any one of expired air, mucosa and skin of the subject. In this configuration, because the acetone concentration value is measured based on the acetone that is released from a subject, it is possible to measure the acetone concentration that is appropriate for determining whether the exercise load of the subject is appropriate or not.

In the exercise load determination method according to one embodiment of the present invention, the output step may output information about an exercise load of exercise to be performed next time as the information based on a determination result. In this configuration, because information about the exercise load of exercise to be performed next time based on a result of determining whether the exercise load of the subject is appropriate or not is output, it is possible to output useful information for the subject.

In the exercise load determination method according to one embodiment of the present invention, the information about an exercise load of exercise to be performed next time may be information about necessity of a change of an exercise load of the exercise performed by the subject. In this case, it is possible to output information that notifies the subject of whether it is necessary to change the current exercise load of exercise.

The exercise load determination method according to one embodiment of the present invention may further include an exercise load acquisition step of acquiring exercise load information being information indicating an exercise load during the exercise, and the determination step may determine whether the exercise load of the exercise is appropriate for the subject based on the comparison result and the exercise load information. In this case, because the determination is made in consideration of the current exercise load, it is possible to make determination by taking the exercise load of the subject into consideration compared with the case of determining whether the exercise load is appropriate based only on the comparison result of the acetone concentration.

In the exercise load determination method according to one embodiment of the present invention, the exercise load information may be information based on at least one of a rating of perceived exertion of the subject and a heart rate of the subject. In this case, it is possible to determine whether the exercise load is appropriate or not by using information about the exercise intensity of the subject as the exercise load information.

In the exercise load determination method according to one embodiment of the present invention, the threshold may be any one of a value based on statistical information, a value based on a weight loss target of the subject, a value based on a past acetone concentration measurement history of the subject, a value based on determination information by an exercise trainer or a doctor a value based on physical condition information of the subject, and a value based on an exercise load of exercise performed and a dietary intake status of the subject before exercise. In this case, because the comparison can be made based on a threshold in accordance with the purpose or the current status of a subject, it is possible to accurately determine whether the exercise load is appropriate or not.

### Advantageous Effects of Invention

According to one embodiment of the present invention, it is possible to easily and accurately determine whether an exercise load is appropriate or not.

### Brief Description of Drawings

Fig. 1 is a view showing a system configuration according to an embodiment of the present invention.
Fig. 2 is a view showing a hardware configuration according to an embodiment of the present invention.
Fig. 3 is a view showing an example of measurement results of breath acetone concentration before exercise, during exercise and after exercise when exercise intensity changes.
Fig. 4 is a view showing an example of measurement results of breath acetone concentration before exercise, during exercise and after exercise with and without dietary intake.
Fig. 5 is a flowchart according to a first embodiment.
Fig. 6 is a flowchart according to a second alternative example.

### Description of Embodiments

Embodiments of the present invention are described hereinafter with reference to the drawings. Note that, where possible, the same elements are denoted by the same reference numerals and redundant description thereof is omitted.

### <First embodiment>

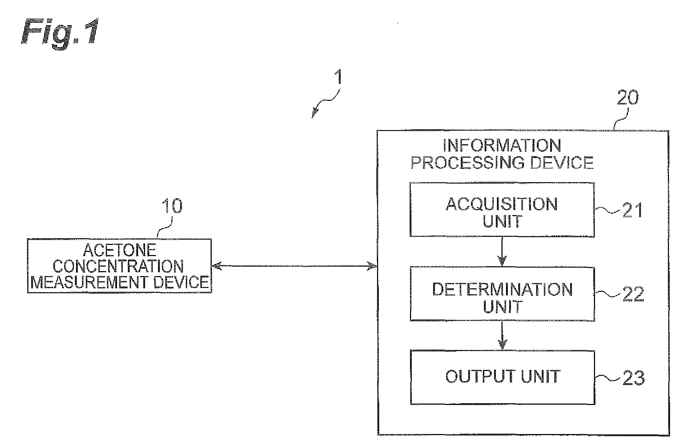

Fig. 1 shows an exercise load determination device 1 according to a first embodiment of the present invention. The exercise load determination device 1 is a device that makes determination about whether an exercise load of the exercise of a subject is appropriate for the subject or not. The term "appropriate" means that an exercise load of the exercise performed by a subject is sufficient to burn fat and matches the athletic ability of the subject, for example. Note that, however, the appropriateness may be defined from another point of view depending on the purpose of the exercise.

As shown in Fig. 1, the exercise load determination device 1 according to this embodiment includes an acetone concentration measurement device 10 and an information processing device 20. The acetone concentration measurement device 10 is a means of measuring the concentration of acetone released from a subject.

The acetone concentration measurement device 10 and the information processing device 20 are connected by wired or wireless communications and can transmit and receive information to and from each other. The acetone concentration measurement device 10 outputs the measured acetone concentration value to the information processing device 20.

The acetone concentration measurement device 10 is portable measuring instrument with which measurements can be carried out easily at a fitness center, home or away from home, for example (cf. e.g., T. Toyooka et al., J. Breath Res., vol.7, 036005, 2013).

The acetone concentration measurement device 10 includes an expired air detection sensor that detects the expired air of a subject, an acetone detection sensor that detects acetone from the expired air, an acetone concentration calculation circuit that calculates the concentration value of acetone from a detection result of the acetone detection sensor, and a transmitting means that transmits the acetone concentration value calculated by the acetone concentration calculation circuit to the information processing device 20. The acetone concentration measurement device 10 according to the present invention is not limited to have the above configuration, and it may have a configuration that does not include the acetone concentration calculation circuit that calculates the concentration value of acetone from a detection result of the acetone detection sensor, and transmits a detection result of the acetone detection sensor (sensor signal data) to another device such as the information processing device 20.

In the acetone concentration measurement device 10, the expired air detection sensor detects the expired air of a subject, the acetone detection sensor detects acetone from the expired air, the acetone concentration calculation circuit calculates the acetone concentration based on a detection result of acetone, and the transmitting means transmits the acetone concentration value to the information processing device 20. In this manner, the acetone concentration measurement device 10 outputs the concentration value of acetone released from a subject through the expired air of the subject to the information processing device 20. The acetone concentration measurement device 10 measures the concentration value of acetone released from a subject before the exercise of the subject (first acetone concentration value) and the concentration value of acetone released from the subject after a certain period of time from the end of the exercise of the subject (at the timing when fat burning is reflected on the acetone concentration value) (second acetone concentration value).

To be specific, a subject blows expired air to the acetone concentration measurement device 10 before exercise. In response thereto, the acetone concentration measurement device 10 calculates the first acetone concentration value from the expired air and stores the first acetone concentration value into a storage means included in the acetone concentration calculation circuit. Alternatively, the acetone concentration measurement device 10 may transmit the calculated first acetone concentration value to the information processing device 20 without storing it into the acetone concentration calculation circuit.

Then, when a specified period (a set period of time) has elapsed after the subject ends the exercise, the subject blows expired air to the acetone concentration measurement device 10 again, and in response thereto, the acetone concentration measurement device 10 calculates the second acetone concentration value from the expired air and transmits the first acetone concentration value and/or the second acetone concentration value to the information processing device 20.

In the case where the acetone concentration measurement device 10 is portable, a subject can easily carry the acetone concentration measurement device 10, and it is possible to measure the acetone concentration value not only before and during the exercise of the subject but also after the lapse of a specified period (a set period of time) from the end of the exercise, without need for a complicated operation (for example, the subject goes out of the way to the acetone concentration measurement device connected to an exercise machine).

The information processing device 20 performs information processing based on the information received from the acetone concentration measurement device 10 and thereby makes determination about whether an exercise load of the exercise of a subject is appropriate for the subject or not. To be specific, the information processing device 20 corresponds to a device such as server device, a work station, a PC (Personal Computer) and a portable terminal. In the information processing device 20, each function, which is described later, is implemented as a result that those components operate by a program or the like. Note that, although the information processing device 20 is one device in this embodiment, it may be an information processing system where a plurality of information processing devices (for example, a portable terminal and a server device etc.) are connected with each other via a network.

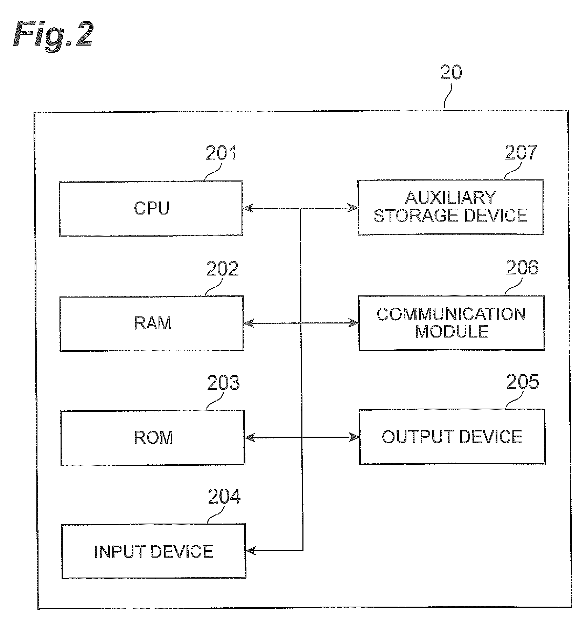

Fig. 2 shows a hardware configuration of the information processing device 20. As shown in Fig. 2, the information processing device 20 is physically configured as a computer system that includes one or a plurality of CPU 201, a RAM 202 and a ROM 203 which are a main storage device, an input device 204 such as a keyboard and a mouse, an output device 205 such as a display, a communication module 206 which is a data transmitting and receiving device such as a network card, an auxiliary storage device 207 such as a semiconductor memory and the like. The information processing device 20 is implemented by loading given computer software onto hardware such as the CPU 201 or the RAM 202 shown in Fig. 2, making the input device 204, the output device 205 and the communication module 206 operate under control of the CPU 201, and performing reading and writing of data in the RAM 202 and the auxiliary storage device 207.

Referring back to Fig. 1, the information processing device 20 includes an acquisition unit 21 (first acetone concentration acquisition means and second acetone concentration acquisition means) that acquires various information, which is described later, a determination unit 22 (determination means) that makes determination based on information acquired by the acquisition unit 21, and an output unit 23 (output means) that outputs a determination result by the determination unit 22.

The acquisition unit 21 acquires the first acetone concentration value and the second acetone concentration value by receiving them from the acetone concentration measurement device 10. Further, the acquisition unit 21 acquires an arbitrary exercise load value (for example, exercise intensity and exercise time) input by a subject or the like using the input device 204 and information about the dietary intake status of the subject (for example, information indicating whether the subject is fasting or not). The exercise load value may be input after exercise (the exercise load value of the exercise already performed) or may be input before exercise (the exercise load value of the exercise to be performed). The acquisition unit 21 outputs the timing of measuring the second acetone concentration value based on the exercise load value and the information about the dietary intake status of the subject.

The acquisition unit 21 stores information in which the exercise load value, the information about the dietary intake status of the subject, and the information about the timing of measuring the second acetone concentration value are associated with one another, and specifies the information about the timing of measuring the second acetone concentration value corresponding to the exercise load value and the information about the dietary intake status of the subject, and outputs the information about the timing of measuring the second acetone concentration value. The information about the timing of measuring the second acetone concentration value is information about the timing when fat burning is reflected on acetone concentration value, and it is the elapsed time from the end of exercise, for example.

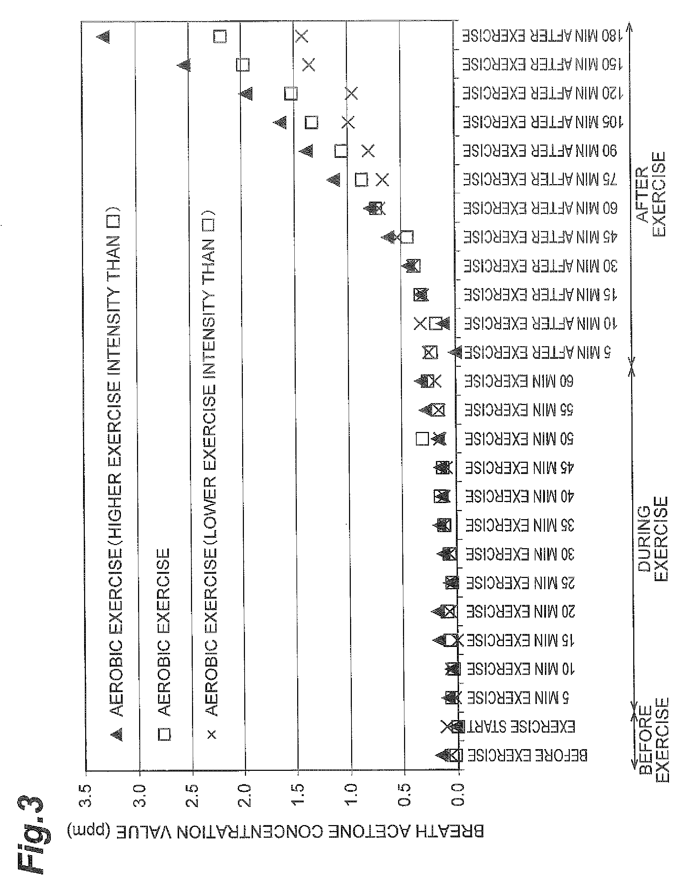

Fig. 3 shows an example of measurement results, for each exercise intensity, of the breath acetone concentration from before to after exercise when a subject performs bicycle ergometer exercise in the state of not fasting. Fig. 3 shows the average of variation of the breath acetone concentration due to the exercise by eight healthy adults being tested in the case where the exercise intensity is high (for example, when exercise is performed for 60 minutes at an exercise load of AT value+1/3×RC value), the case where the exercise intensity is moderate (for example, when exercise is performed for 60 minutes at an exercise load of AT value) and the case where the exercise intensity is low (for example, when exercise is performed for 60 minutes at an exercise load of AT value-1/3×RC). In the graph of Fig. 3, the horizontal axis indicates the time axis from before the start of exercise to 180 minutes after the end of exercise, and the vertical axis indicates the breath acetone concentration value of the subject.

As shown in Fig. 3, in the case of the moderate exercise intensity, the time when the acetone concentration value becomes higher than 1.0ppm is 90 minutes after the exercise on average, whereas, in the case of the high exercise intensity, the time when the acetone concentration value becomes higher than 1.0ppm is 75 minutes after the exercise on average. Further, in the case of the low exercise intensity, the time when the acetone concentration value becomes higher than 1.0ppm is 150 minutes after the exercise on average.

Thus, a specified time from the end of exercise (the measurement timing of the second acetone concentration value) when the burning of the fat of the subject is noticeably reflected on the breath acetone concentration varies to be shorter or longer in accordance with the level of an exercise load.

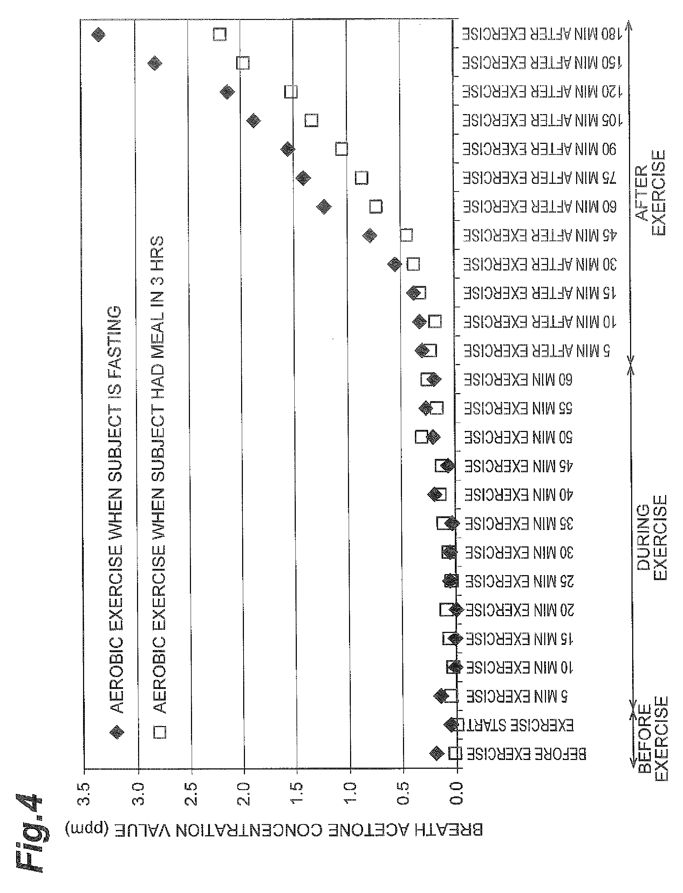

Fig. 4 shows an example of measurement results of the breath acetone concentration before exercise, during exercise and after exercise with and without dietary intake. Fig. 4 shows the average of variation of the breath acetone concentration value due to the bicycle ergometer exercise by eight healthy adults being tested in the case of the fasting state where six hours or more have elapsed from the last meal and the case where one had a meal within three hours before the start of measurement. In the graph of Fig. 4, the horizontal axis indicates the time axis from before the start of exercise to 180 minutes after the end of exercise, and the vertical axis indicates the breath acetone concentration value of the subject.

As shown in Fig. 4, in the case where the subject performs the bicycle ergometer exercise at an exercise load of AT value for 60 minutes when the subject is fasting, the time when the acetone concentration value becomes higher than 1.0 ppm is 60 minutes after the exercise on average, whereas, in the case where the subject had a meal within three hours before the start of measurement, it is 90 minutes after the exercise.

Thus, a specified time from the end of exercise (the measurement timing of the second acetone concentration value) when the burning of the fat of the subject is noticeably reflected on the breath acetone concentration value varies to be shorter or longer in accordance with the dietary intake status before the start of measurement. Note that, as shown in Figs. 3 and 4, the fat burning during exercise is not noticeably reflected on the breath acetone concentration, and the variation of the concentration is only a little in any conditions with different exercise loads and dietary intake status.

Based on the above, the acquisition unit 21 stores information about the timing when fat burning is noticeably reflected on the acetone concentration value (for example, information about a recommended measurement time after the end of exercise) which is determined based on the exercise load of the exercise performed or the dietary intake status before exercise, and outputs to display the information about the timing at a point of time after the exercise of the subject (for example, at a point of time when the exercise load of the exercise performed is input after the exercise of the subject) and notifies the subject of the timing of measuring the acetone concentration value after the end of exercise. It then prompts the subject to measure the second acetone concentration value based on that tiring. The acquisition unit 21 can thereby acquire, from the acetone concentration measurement device 10, the second acetone concentration value that is measured the timing when fat burning is noticeably reflected on the acetone concentration value.

The determination unit 22 acquires the first acetone concentration value, the second acetone concentration value and the exercise load value from the acquisition unit 21, calculates the variation from the first acetone concentration value to the second acetone concentration value, compares the variation with a preset threshold, and determines whether the exercise load of the exercise performed by the subject is appropriate for that subject or not based on the comparison result and the exercise load value. The variation is a difference value between the first acetone concentration value and the second acetone concentration value (which is a value obtained by subtracting the first acetone concentration value from the second acetone concentration value in this example). Note that it may be any value that represents a result of comparison, such as a ratio, for example. Specifically, it may be any value that compares the first acetone concentration value and the second acetone concentration value (comparison value).

The threshold that is preset in the determination unit 22 is the variation of the acetone concentration value at which a certain degree of fat burning effect is expected, and it may be a value on the basis of statistical information, a value determined by a subject in accordance with his/her weight loss target, a value determined based on the past acetone concentration measurement history of a subject, a value determined under the judgment of a third person like an exercise trainer or a doctor, or an arbitrary value according to the physical condition or the like. To be specific, the value on the basis of statistical information is the average of the acetone concentration variation in a subject who has successfully lost weight, for example. Further, when the weight loss target of a subject is BMI (body mass index) 22, for example, the average of the acetone concentration variation of a person who keeps the BMI can be a preset threshold. Then, the average of the acetone concentration variation of that subject can be found by referring to the past acetone concentration measurement history of the subject, and a value larger than that average (for example, twice the average) can be a preset threshold. Note that a threshold may be arbitrarily set based on the knowledge and experience of a specialist like an exercise trainer or a doctor, and the set threshold may be fine-adjusted depending on the physical condition of a subject on the day of exercise.

Then, the determination unit 22 determines output information based on a result of the determination. For example, the determination unit 22 determines to output information about the next exercise load of a subject based on the determination result (or as the determination result). To be specific, the determination unit 22 determines to output information about increase or decrease of the exercise load in comparison with the current exercise load (for example, information indicating to increase the exercise load of the next time to be higher than that of this time) as the information about the next exercise load. Then, the determination unit 22 may determine to output information about increase or decrease of the exercise intensity only, information about increase or decrease of the exercise time only, or information about increase or decrease of both the exercise intensity and the exercise time, as the information about increase or decrease of the exercise load. In this case, the determination unit 22 determines information to be output in accordance with a divergence between the measured acetone concentration variation and the threshold or the like. A specific way of determining output information by the determination unit 22 is described later.

The output unit 23 outputs the information determined by the determination unit 22. Further, when the acetone concentration measurement device 10 has a display means (for example, a display), the output unit 23 may transmit information on the basis of the determination result so that the display means of the acetone concentration measurement device 10 outputs it. The output unit 23 may transmit the information to a portable terminal of a subject, an exercise trainer, a doctor or the like through a network so that the information on the basis of the determination result is displayed on the display means of the portable terminal. In this manner, by displaying the determination result on various devices, the exercise load determination device 1 can achieve "visualization" of fat burning effect of the exercise to the subject.

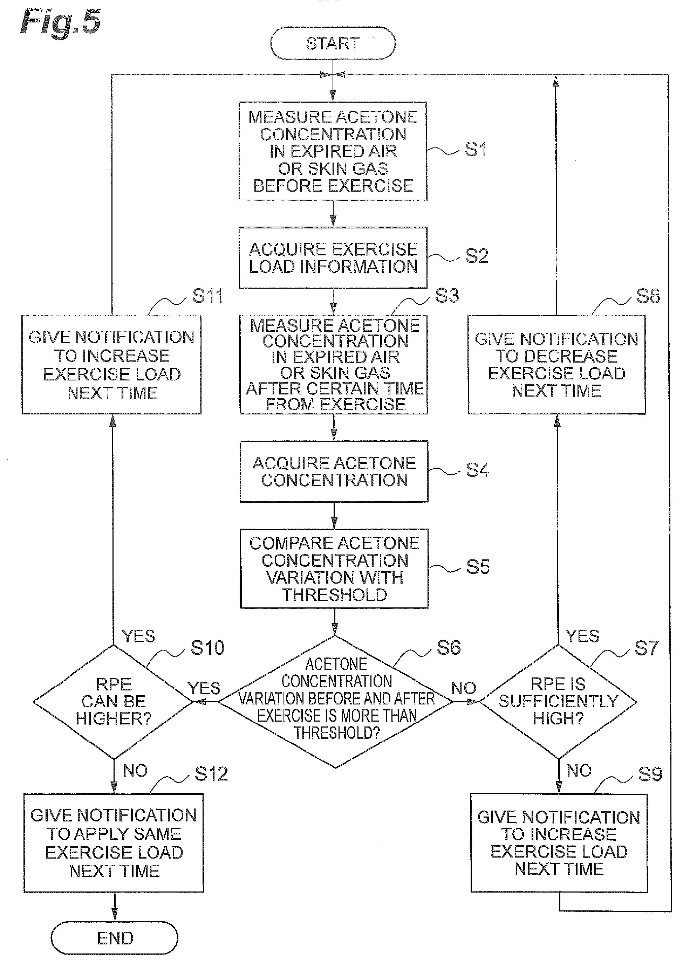

A process and an operation (exercise load determination method) performed in the exercise load determination device 1 according to this embodiment are described hereinafter with reference to Fig. 5. In the exercise load determination device 1 according to this embodiment, the acetone concentration value (the first acetone concentration value) in the expired air of a subject is measured by the acetone concentration measurement device 10 before the exercise of the subject (Step S1, first measurement step). Then, the subject performs arbitrary exercise.

After the exercise of the subject, the acquisition unit 21 receives input of information about the exercise load of the subject and stores the information about the exercise load (Step S2, exercise load acquisition step). In this embodiment, as the information about the exercise load of the subject, the current rating of perceived exertion (RPE value) is input. Note that, when the subject performs the process of the exercise load determination method for the first time, it is preferred to begin with a low exercise load (for example, the RPE value (Borg scale) indicating an exercise load is about 10); however, one may begin with an exercise load corresponding to an arbitrary value. The RPE is the way of estimating a rating of perceived exertion by the determination of a subject among fifteen levels from 6 to 20 (which is called Borg scale) to indicate how tough the current exercise is based on the subjective view of the subject. When the value of RPE is large, it means that the exercise load which the subject perceives as tough is imposed, and when the value of RPE is small, it means that the subject does not perceive the exercise as too tough.

Note that, as another scale indicating the exercise intensity, METs (Metabolic equivalents), which is 1 in the resting state, can be used, and the intensity list from daily life activities to vigorous exercises is issued by the Incorporated Administrative Agency, National Institute of Health and Nutrition. Besides, as a measure of exercise intensity, an index on the basis of heart rate (HR) (for example, a heart rate during exercise/maximum heart rate) or the like may be used, for example.

Then, after a specified period has elapsed from the exercise of the subject, the acetone concentration value (the second acetone concentration value) in the expired air of the subject is measured by the acetone concentration measurement device 10 (Step S3, second measurement step). Note that the acetone concentration measurement device 10 stores the acetone concentration value measured in Step S1 and Step S3 in a storage means. Then, when the first acetone concentration value and the second acetone concentration value are transmitted from the acetone concentration measurement device 10, the acquisition unit 21 of the information processing device 20 acquires the first acetone concentration value and the second acetone concentration value (Step S4, first acetone concentration acquisition step and second acetone concentration acquisition step). The determination unit 22 of the information processing device 20 compares the variation between the acetone concentration value before the exercise and the acetone concentration value after the lapse of a specified period from the exercise acquired by the acquisition unit 21 with a threshold (Step S5, determination step).

Then, the determination unit 22 determines whether the variation of the acetone concentration value before and after the exercise is larger than a predetermined threshold or not (Step S6, determination step), and further determines whether the exercise load of the exercise performed by the subject is appropriate for that subject or not based on a comparison result between the variation and the threshold and the RPE value by referring to the current RPE value to thereby determine the output information (Step S7, Step S10, determination step). The output unit 23 outputs information about the exercise load to be performed by the subject next time as the output information determine by the determination unit 22 based on the comparison result and the current RPE value. To be specific, the determination unit 22 and the output unit 23 perform the following output processing steps (1) to (4) based on the determination result in Step S6 and the current RPE value.
(1) When "threshold" ≤ "measured acetone concentration variation" and when there is no remaining capacity for RPE (Yes in Step S6; No in Step S10)
   In this case, because the acetone concentration value variation is equal to or more than a threshold, a certain degree of fat burning effect is expected. Further, because it is unable to keep up with the higher exercise load in terms of RPE also, the current exercise load is estimated to be appropriate. Based on this estimation, the determination unit 22 determines that the exercise load of the exercise performed by the subject is appropriate for the subject, and determines to output information indicating that the same exercise load as in this time is to be applied next time. Then, the output unit 23 outputs the output information determined by the determination unit 22 (Step S12, output step), and the process ends. The case where there is no remaining capacity for RPE is when the RPE value is equal to or larger than the appropriate exercise value (about RPE 12 to 14) of the subject which is prestored in the determination unit 22.
(2) When "threshold" ≤ "measured acetone concentration value variation" and when there is remaining capacity for RPE (Yes in Step S6; Yes in Step S10)
   In this case, because the acetone concentration value variation is equal to or more than a threshold, a certain degree of fat burning effect is expected. However, because there is remaining capacity for RPE, it is considered that the current exercise load is not necessarily appropriate. Thus, the determination unit 22 determines to output information, indicating to increase the exercise load of the next time to be higher than this time. Then, the output unit 23 outputs the output information determined by the determination unit 22 (Step S11, output step), and the process returns to Step S1. The case where there is remaining capacity for RPE is when the RPE value is smaller than the appropriate exercise value (about RPE 12 to 14) of the subject.
(3) When "threshold" > "measured acetone concentration value variation" and when RPE is sufficiently high (No in Step S6; Yes in Step S7)
   In this case, because the acetone concentration value variation is less than a threshold, and no sufficient fat burning effect can be expected. RPE is sufficiently high, and it is estimated that the subject is in the overload state. Thus, the determination unit 22 determines the exercise load of the exercise performed by the subject is not appropriate for the subject, and determines to output information indicating to decrease the exercise load of the next time to be lower than this time. Then, the output unit 23 outputs the output information determined by the determination unit 22 (Step S8, output step), and the process returns to Step S1. The case where RPE is sufficiently high is when the RPE value exceeds the appropriate exercise value (about RPE 12 to 14) of the subject which is prestored in the determination unit 22.
(4) When "threshold" > "measured acetone concentration variation" and when RPE is not high (No in Step S6; No in Step S7)
   In this case, because the acetone concentration value variation is less than a threshold, no sufficient fat burning effect can be expected and further RPE can be still higher, the determination unit 22 determines the exercise load of the exercise performed by the subject is not appropriate for the subject, and determines to output information indicating to increase the exercise load of the next time to be higher than this time. Then, the output unit 23 outputs the output information determined by the determination unit 22 (Step S9, output step), and the process returns to Step S 1. The case where RPE is not high is equal to the case where there is remaining capacity for RPE.

In this manner, according to the exercise load determination method described above, information that encourages the adjustment of an exercise load is output to prompt a subject to repeat the adjustment of the exercise load several times, thereby achieving the most appropriate exercise load. Note that, even when the most appropriate exercise load is determined, the exercise load determination method according to the present invention may be applied again when performing the exercise next time. In other words, the acetone concentration before and after exercise may be measured every time performing the exercise. This is because the tolerance to the exercise load increases by repeating the same exercise, which makes it difficult to burn the fat without knowing, and further the dietary intake status and the physical conditions are often different day by day.

Note that, although RPE is used in the above-described example, it is not limited thereto, and an index on the basis of the above-described HR may be used instead of the RIPE value, or an index on the basis of HR may be used in addition to the RPE value.

### <alternative example 1>

An alternative example 1 of the present invention is described hereinafter. In the above-described embodiment, the case where an arbitrary value of exercise load is input is described; however, in the alternative example 1, the information processing device 20 encourages a subject to perform exercise at a low exercise load before the first exercise (for example, at the measurement of the first acetone concentration value). The low exercise load is an exercise load that is lower than an appropriate exercise value, for example. In this case, because the information processing device 20 begins with a low exercise load and gradually increases the exercise load, the determination on RPE (Steps S7 and S10 in the flowchart of Fig. 5) is not necessary, which enables simplification of the process.

In the alternative example 1, the following processing is performed in the branch of Step S6 in the flowchart of Fig. 5. When the determination unit 22 determines that "threshold" ≤ "measured acetone concentration variation", the determination unit 22 determines that the exercise load of the exercise performed by the subject is appropriate for the subject, and determines to output information indicating that the same exercise load as in this time is to be applied next time. Then, the output unit 23 outputs the output information determined by the determination unit 22. This is because the acetone concentration variation is equal to or more than a threshold, a certain degree of fat burning effect is expected, and it is thus estimated that the current exercise load is appropriate.

On the other hand, when the determination unit 22 determines that "threshold" > "measured acetone concentration variation", the determination unit 22 determines that the exercise load of the exercise performed by the subject is not appropriate for the subject, and determines to output information indicating to increase the exercise load of the next time to be higher than this time. Then, the output unit 23 outputs the output information determined by the determination unit 22. This is because the acetone concentration variation is less than a threshold, and no sufficient fat burning effect can be expected.

### <Alternative example 2>

An alternative example 2 is described hereinafter. In the alternative example 2, when the variation is more than a threshold, information indicating to decrease the exercise load is output. It is thereby possible to avoid excessive exercise of a subject, and modify the exercise load so that the measured acetone concentration variation becomes substantially the same as the threshold.

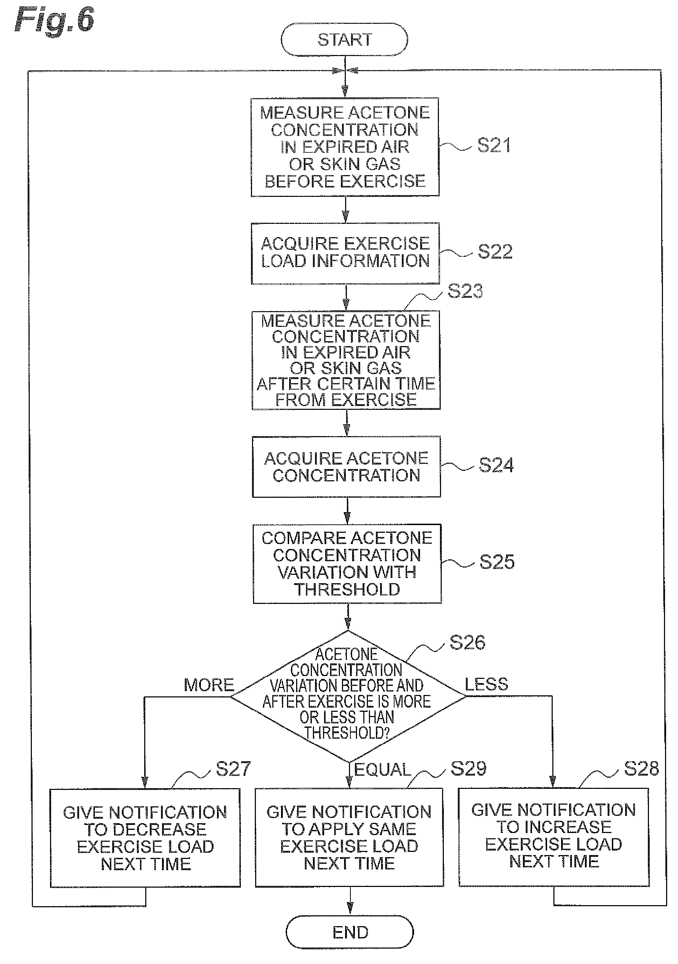

Fig. 6 shows a flowchart of a process of the alternative example 2. The processing of Steps S21 to S25 is the same as the processing of Steps S1 to S5 of the flowchart of Fig. 5, respectively, and thus not redundantly described.

After Step S25, the determination unit 22 determines a comparison result between the acetone concentration value variation before and after exercise and a predetermined threshold (Step S26, determination step). When the determination unit 22 determines that the acetone concentration variation before and after exercise is more than the equal range of the predetermined threshold, it determines that the exercise load of the exercise performed by the subject is not appropriate for the subject, and determines to output information indicating to decrease the exercise load next time. The equal range of the threshold indicates a certain range including the threshold, and information about this range is stored in the determination unit 22. The output unit 23 outputs the output information determined by the determination unit 22 (Step S27, output step), and the process returns to Step S21. The information processing device 20 can thereby encourage the subject to avoid excessive exercise.

In Step S26, when the determination unit 22 determines that the acetone concentration value variation before and after exercise is less than the equal range of the predetermined threshold, it determines that the exercise load of the exercise performed by the subject is not appropriate for the subject, and determines to output information indicating to increase the exercise load next time. The output unit 23 outputs the output information determined by the determination unit 22 (Step S28, output step), and the process returns to Step S21. The information processing device 20 can thereby encourage the subject to change the exercise load to enhance the fat burning effect.

In Step S26, when the determination unit 22 determines that the acetone concentration value variation before and after exercise is equal to the threshold (included in the equal range), it determines that the exercise load of the exercise performed by the subject is appropriate for the subject, and determines to output information indicating that the same exercise load as in this time is to be applied next time. The output unit 23 outputs the output information determined by the determination unit 22 (Step S29, output step) and the process ends.

### <Alternative example 3>

In the above-described embodiment, the acquisition unit 21 specifies information about the measurement timing after exercise when fat burning is noticeably reflected on the breath acetone concentration based on the load of exercise performed by a subject and information about the dietary intake status before exercise. Then, the acquisition unit 21 outputs the specified measurement timing information and prompts the subject to measure the acetone concentration value after exercise based on the output measurement timing information. In the alternative example 3, thresholds in accordance with the load of exercise performed and information about the dietary intake status before exercise at the fixed measurement timing (for example, 60 minutes after the end of exercise) are stored in the acquisition unit 21, and a threshold to be used for the comparison with the variation by the determination unit 22 is determined based on the load of exercise performed and the information about the dietary intake status before exercise that are input and received.

Specifically, the timing of measuring the acetone concentration value after exercise is fixed regardless of the conditions such as the exercise load and the dietary intake status, and a predetermined threshold is varied instead according to the conditions. For example, in the case of applying a high exercise load like AT value+1/3)×RC value or performing exercise in the fasting state, the acquisition unit 21 determines a threshold to be a larger value than the predetermined standard threshold, and in the case of applying a low exercise load like AT value-1/3×RC value or performing exercise not in the fasting state, the acquisition unit 21 determines a threshold to be a smaller value than the predetermined standard threshold

### (Advantageous effects)

Advantageous effects are described hereinbelow. The acquisition unit 21 acquires the first acetone concentration value, which is the concentration of acetone released from a subject before the exercise of the subject, and the second acetone concentration value, which is the concentration of acetone released from the subject after a certain period of time from the end of the exercise of the subject, and the determination unit 22 calculates a comparison value between the first acetone concentration value and the second acetone concentration value, compares the comparison value with a preset threshold, determine whether the exercise load of the exercise is appropriate for the subject or not, and outputs information based on the determination result.

According to the above-described exercise load determination method, because the determination is made using the acetone concentration value after a specified period of time has elapsed from the end of the exercise of the subject, which is the timing when fat burning is noticeably reflected on the acetone concentration, it is possible to accurately determine whether the exercise load is appropriate or not. Further, because the determination is made based on the acetone concentration value measured before and after the exercise of the subject, it is possible to easily determine whether the exercise load is appropriate or not compared with the case of performing a cardiopulmonary exercise test.

The acetone concentration measurement device 10 measures the first acetone concentration value and the second acetone concentration value from a subject, and the acquisition unit 21 acquires the first acetone concentration value and the second acetone concentration value. In this case, it is possible to reliably acquire the first acetone concentration value and the second acetone concentration value.

The output unit 23 outputs information about the exercise load of exercise to be performed next time as information based on the determination result. Because the output unit 23 outputs information about the exercise load to be performed next time by a subject and thereby outputs information about the exercise load of exercise to be performed next time based on a result of determining whether the exercise load of the subject is appropriate or not, it is possible to output useful information for the subject.

In the above-described embodiment, the information about the exercise load of exercise to be performed next time is information about the necessity of a change of the exercise load of exercise performed by a subject (for example, information whether to increase the exercise load next time). In this case, it is possible to output information that notifies the subject of whether it is necessary to change the current exercise load of exercise.

Exercise load information (for example, RPE value), which is information indicating the exercise load during exercise, is acquired, and it is determined whether the exercise load of exercise is appropriate for the subject based on the comparison result and the exercise load information. In this case, because the determination is made in consideration of the current exercise load, it is possible to make determination by taking the exercise load of the subject into consideration compared with the case of determining whether the exercise load is appropriate based only on the comparison result of the acetone concentration.

The exercise load information is information that is based on at least one of the rating of perceived exertion (RPE value) of a subject and the heart rate of a subject. In this case, it is possible to determine whether the exercise load is appropriate or not by using information about the exercise intensity of the subject as the exercise load information.

The timing of measuring the second acetone concentration value is determined depending on the exercise performed or the information about the dietary intake condition of a subject. In this case, the second acetone concentration value at the timing when fat burning is noticeably reflected on the acetone concentration value can be acquired.

The threshold for variation may be any one of a value on the basis of statistical information, a value on the basis of weight loss target of a subject, a value on the basis of the past acetone concentration measurement history of a subject, a value on the basis of determination information by an exercise trainer or a doctor, a value on the basis of physical condition information of a subject, and a value on the basis of the exercise load of exercise performed and the dietary intake status of a subject before exercise. In this case, because the comparison can be made based on a threshold in accordance with the purpose or the current status of a subject, it is possible to accurately determine whether the exercise load is appropriate or not.

### (Other embodiments)

Although the case where the acetone concentration measurement device 10 measures acetone from expired air is described in the above embodiment, skin acetone that is naturally released from the skin or mucosa may be measured. Further, a part where skin acetone is released is not particularly limited, and it may be any part as long as skin acetone is released. In this manner, because the acetone concentration measurement device 10 measures the acetone concentration value based on the gas component that is released from a subject, it is possible to measure the acetone concentration that is appropriate for determining whether the exercise load of the subject is appropriate or not. Note that, it is known that there is correlation between breath acetone concentration value and skin acetone concentration value (cf. e.g., C. Turner et al., Rapid Commun. Mass Spectrom., viol.22, pip.526-532, 2008).

Although the case where the concentration value of acetone is measured using the portable acetone concentration measurement device 10 is described in the above embodiment, the acetone concentration may be measured using other various equipment capable of measuring the acetone concentration. For example, another semiconductor gas sensor, a gas chromatography device, an ion mobility spectrometry device or the like may be used.

Although the case where the determination unit 22 determines information about the exercise load of exercise to be performed next time and the output unit 23 outputs the information determined by the determination unit 22 is described in the above embodiment, the determination unit 22 may compare the variation with a threshold, determines whether the exercise load of the exercise performed by a subject is appropriate for that subject, and determine to output information about a determination result (for example, information indicating that the exercise load of the exercise performed by a subject is appropriate for that subject), and the output unit 23 may output the information determined by the determination unit 22.

Although ergometer is taken as an example of exercise in the above-described embodiment, other exercise may be performed, such as treadmill, pressure training or hydraulic pressure training, for example. Further, the exercise may be jogging, walking, cycling, swimming, muscle exercise or the like. Note that the type of exercise to be performed may be decided by a subject or may be decided by an exercise trainer or a doctor. Further, a plurality of types of exercise may be performed in combination.

Although the case where information about the acetone concentration value is transmitted from the acetone concentration measurement device 10 to the information processing device 20 is described in the above embodiment, the information processing device 20 may request the acquisition of the acetone concentration value to the acetone concentration measurement device 10 without transmitting the acetone concentration value spontaneously from the acetone concentration measurement device 10 to the information processing device 20.

In the above-described embodiment, the exercise load determination device 1 is separated into the acetone concentration measurement device 10 and the information processing device 20; however, the acetone concentration measurement device 10 and the information processing device 20 may be integrated into one device. Further, the acetone concentration measurement device 10 and some functions of the information processing device 20 may be integrated into one device. For example, the acetone concentration measurement device 10 and the output unit 23 may be integrated into one device. Further, the functions of the information processing device 20 may be separated into a plurality of devices. For example, the information processing device 20 may be separated into a device including the acquisition unit 21 and the determination unit 22 and a device including the output unit 23.

### Reference Signs List

1... exercise load determination device, 10... acetone concentration measurement device, 20... information processing device, 21...acquisition unit, 22... determination unit, 23...output unit, 201...CPU, 202...RAM, 203...ROM, 204...input device, 205...output device, 206...communication, module, 207...auxiliary storage device

## Claims

1. An exercise load determination method comprising:
a first acetone concentration acquisition step of acquiring first acetone concentration value being concentration value of acetone released from a subject before exercise of the subject;
a second acetone concentration acquisition step of acquiring second acetone concentration value being concentration value of acetone released from the subject after a specified period of time from an end of the exercise by the subject;
a determination step of calculating a comparison value between the first acetone concentration value acquired in the first acetone concentration acquisition step and the second acetone concentration acquired value in the second acetone concentration acquisition step, comparing the comparison value with a preset threshold, and determining whether an exercise load of the exercise is appropriate for the subject; and
an output step of outputting information based on a determination result in the determination step.

2. The exercise load determination method according to Claim 1, wherein the specified period of time is set in accordance with the exercise performed or information about a dietary intake condition of the subject.

3. The exercise load determination method according to Claim 1 or 2, further comprising:
a first measurement step of measuring the first acetone concentration value from the subject; and
a second measurement step of measuring the second acetone concentration value from the subject, wherein
the first acetone concentration acquisition step acquires the first acetone concentration value measured in the first measurement step, and
the second acetone concentration acquisition step acquires the second acetone concentration value measured in the second measurement step.

4. The exercise load determination method according to any one of Claims 1 to 3, wherein
the first acetone concentration value and the second acetone concentration value are concentration value of acetone measured based on a gas component produced from at least any one of expired air, mucosa and skin of the subject.

5. The exercise load determination method according to any one of Claims 1 to 4, wherein
the output step outputs information about an exercise load of exercise to be performed next time as the information based on a determination result.

6. The exercise load determination method according to Claim 5, wherein
the information about an exercise load of exercise to be performed next time is information about necessity of a change of an exercise load of the exercise performed by the subject.

7. The exercise load determination method according to any one of Claims 1 to 5, further comprising:
an exercise load acquisition step of acquiring exercise load information being information indicating an exercise load during the exercise, wherein
the determination step determines whether the exercise load of the exercise is appropriate for the subject based on the comparison result and the exercise load information.

8. The exercise load determination method according to Claim 7, wherein
the exercise load information is information based on at least one of a rating of perceived exertion of the subject and a heart rate of the subject.

9. The exercise load determination method according to any one of Claims 1 to 8, wherein
the threshold is any one of a value based on statistical information, a value based on a weight loss target of the subject, a value based on a past acetone concentration measurement history of the subject, a value based on determination information by an exercise trainer or a doctor, a value based on physical condition information of the subject, and a value based on an exercise load of exercise performed and a dietary intake status of the subject before exercise.

10. An exercise load determination device comprising:
a first acetone concentration acquisition means configured to acquire first acetone value concentration being concentration value of acetone released from a subject before exercise of the subject;
a second acetone concentration acquisition means configured to acquire second acetone concentration value being concentration value of acetone released from the subject after a specified period of time from an end of the exercise by the subject;
a determination means configured to calculate a comparison value between the first acetone concentration value acquired by the first acetone concentration acquisition means and the second acetone concentration value acquired by the second acetone concentration acquisition means, compare the comparison value with a preset threshold, and determine whether an exercise load of the exercise is appropriate for the subject; and
an output means configured to output information based on a determination result by the determination means.
